# EUROPEAN PATENT SPECIFICATION

(11) **EP 2 547 730 B1**
(45) Date of publication and mention of the grant of the patent: **10.07.2019**
(21) Application number: 11756745.3
(22) Date of filing: 09.03.2011
(51) Int. Cl.: C08L 33/06, C08J 3/20, A45D 31/00, A61K 8/34, A61K 8/35, A61K 8/37, A61K 8/87, A61Q 3/02

(54) **METHOD OF PREPARATION OF RADIATION-CURABLE COLORED ARTIFICIAL NAIL GELS**
VERFAHREN ZUR HERSTELLUNG STRAHLUNGSHÄRTBARER GEFÄRBTER KÜNSTLICHER NAGELGELE
PROCÉDÉ DE PRÉPARATION DE GELS COLORÉS DURCISSABLES PAR RAYONNEMENT POUR ONGLES ARTIFICIELS

(30) Priority: 16.03.2010 US 725073
(43) Date of publication of application: 23.01.2013
(73) Proprietor: Mycone Dental Supply Company Inc., Cherry Hill, NJ 08002 (US); Raney, Robert, Newtown Square, PA 19073-3805 (US); Sheran, Kevin M., Philadelphia, PA 19145 (US); Steffier, Larry W., Cherry Hill, NJ 08034 (US); Iannece, Gary, Bordentown, NJ 08505 (US)
(72) Inventor: RANEY, Robert, Newtown Square, PA 19073-3805 (US); SHERAN, Kevin, M., Philadelphia, PA 19145 (US); STEFFIER, Larry, W., Cherry Hill, NJ 08034 (US); IANNECE, Gary, Bordentown, NJ 08505 (US)
(74) Representative: Lavoix
(86) International application number: PCT/US2011/027703
(87) International publication number: WO 2011/115795

(56) References cited:
- WO-A2-2011/011304
- GB-A- 2 452 566
- US-A- 3 896 014
- US-A- 4 649 045
- US-A- 4 682 612
- US-A- 5 133 966
- US-A- 5 407 666
- US-A1- 2002 010 226
- US-B2- 6 803 394

## Description

### BACKGROUND OF THE INVENTION

Benefit of U.S. Non-Provisional Application Serial No. 12/725,073, filed March 16, 2010 is claimed.

This invention relates to the field of radiation-curable gels useful for cosmetic adornment of natural nails, artificial fingernails, toenails and artificial nail extensions.

The use of radiation-curable gels in formation of nail enhancements or artificial nails has been an important part of the cosmetic industry since it was first introduced. US Pat. 4,682,612, describing the use of actinic radiation-curable compositions suitable for preparation of artificial nails, is representative of this technology.

Ultra-violet radiation (UV) is the most conventional form of radiation used to cure gels in this art, however, visible light curing systems are also known. UV-curable gels are most typically applied by professional nail technicians. Such UV-curable gels are usually composed of acrylic or methacrylic monomers and oligomers in a gel-like state that requires curing under a UV lamp. Such nail finishes can be applied directly to natural fingernails or toenails, or alternatively can be applied to nail extensions bonded to fingernails. In many cases, the artificial nails are coated with conventional nail polish after they are cured.

In order to avoid the need to coat the artificial nails or natural nails with conventional nail polish, in more recent years, the preparation of gels containing colorants, particularly pigments, has become known in this art. However, the previously suggested processes used to prepare such colored gels have several disadvantages. One such process, the direct of addition of pigment powders, is described in US Pat. Pubs. US2006/0283720 and US2010/0008876. The direct use of pigment powders brings with it the disadvantages of difficulty in handling, inconsistent dispersion leading to poor color control, the need for high shear mixing or milling full batches of material, and the need for expensive large scale equipment. The direct mixing of all components including pigment powders in a suitable solvent is described in US Pat. 5.985,951 which brings the same disadvantages. As an alternative to incorporating pigments per sec a gel composition combined with commercial nail polish is described in US Pat. 6,803,394.
In this patent, commercial nail polish is added to a UV-curable artificial nail gel by the applicator as a means for coloring the gel and resultant artificial nails.

The use of commercial nail polish is undesirable in that it limits the depth of color that can be achieved in a given coating weight. Thus, compared to the commercial nail polish itself, a lower color density will be achieved and attempts to increase the amount of nail polish will lead to systems which give poor curing characteristics or discomfort due to the thick coating required to provide the desired color depth.

It has been a long felt need in this art to provide integrally colored artificial nails which have a reproducible, predictable color when cured.

There is also a need for an improved method to produce color containing UV-curable gels which give high color density.

There is also a need for an alternative to directly adding pigments to artificial nail gels to avoid the requirement of applying high shear to the gels in order to effectively incorporate the pigments.

### SUMMARY OF THE INVENTION

These objectives, and others as will become apparent from the following description, are achieved by the present invention which comprises in one aspect a method of preparing colored UV-curable compositions useful for adornment of natural and artificial nails and artificial nail extensions comprising dispersing a pigment in an organic liquid to form a pigment concentrate, the organic liquid comprising one or more organic chemicals selected from solvents, ethylenically unsaturated monomers, and ethylenically unsaturated oligomers, and mixing the dispersed pigment concentrate with a radiation-curable nail gel composition comprising one or more ethylenically unsaturated monomers, one or more ethylenically unsaturated oligomers, or mixtures thereof, and a photoinitiator, wherein the concentration of pigment in the pigment concentrate is 10 to 50%.
It is also disclosed a method for preparing high color density gels without the use of high shear by introducing the pigment in the form of a homogeneously pre-dispersed concentrate.

It is also disclosed an artificial nail gel composition prepared by the method of the invention.

It is further disclosed an artificial nail prepared by curing under actinic radiation an artificial nail gel composition of the invention.
It is also disclosed a highly colored UV-curable artificial nail gel prepared by such method.

### DETAILED DESCRIPTION OF PREFERRED EMBODIMENTS

According to the invention, high density color gels can be successfully prepared using flowable pigment concentrates without the use of high shear in the production of the final gel. The concentrates can be added to gel formulations including commercially available nail gels without the use of high shear conditions to give suitable colored gels for nails.

The UV-curable artificial nail gels can comprise a wide variety of compounds containing one or more radical polymerizable unsaturated double bond. Typical examples include esters of acylic and methacrylic acid, herein termed (meth)acrylic ester. Specific but not limiting examples of mono (meth)acryloyl esters include methyl (meth)acrylate, ethyl (meth)acrylate hydroxypropyl (meth)acrylate, ethyl (meth)acrylate , butyl (meth)acrylates, hydroxy ethyl (meth)acrylates, butoxyethyl (meth)acrylate, diethylaminoethyl (meth)acrylate, 2- ethylhexyl (meth)acrylate, ethoxyethyl (meth)acrylate, t-butyl aminoethyl (meth)acrylate, methoxyethylene glycol (meth)acrylate, phosphoethyl (meth)acrylate, methoxy propyl (meth)acrylate, methoxy polyethylene glycol(meth)acrylate, phenoxyethylene glycol (meth)acrylate, phenoxypolyethylene glycol (meth)acrylate, 2-hydroxy-3-phenoxypropyl (meth)acrylate, 2-(meth)acryloxyethylsuccinic acid, 2-(meth)acryloylethylphthalic acid, 2-(meth)acryloyloxypropylphthalic acid, stearyl (meth)acrylate, isobornyl (meth)acrylate, 3-chloro-2-hydroxypropyl (meth)acrylates, tetrahydrofufuryl (meth)acrylate, (meth)acrylamides and allyl monomers. Specific but not limiting examples of difunctional methacryloyl esers include 1.4 butane diol di(meth)acrylate, 1,6 hexanediol di(meth)acrylate, 1,9 nonanediol di(meth)acrylate, 1,10 decanediol di(meth)acrylate, neopentyl glycol di(meth)acrylate, 2-methyl¬1.8-octane diol di(meth)acrylate, glycerin di(meth)acrylate, ethylene glycol di(meth)acrylate, triethylenglycol di(meth)acrylate, polyethylene glycol di(meth)acrylate, propylene glycol di(meth)acrylate, polypropylene glycol di(meth)acrylate, ethoxylated propylene glycol di(meth)acrylate, ethoxylated polypropylene glycol di(meth)acrylate, polyethoxypropoxy di(meth)acrylate, ethoxylated bisphenol A di(meth)acrylate, propoxylated bisphenol A di(meth)acrylate, propoxylated ethoxylated bisphenol A di(meth)acrylate, bisphenol-A glycidyl dimethacrylate, tricyclodecanedimethanol di(meth)acrylate, glycerin di(meth)acrylate, ethoxylated glycerin di(meth)acrylate, bis acrylamides, bis allyl ethers and allyl (meth)acrylates. Examples of tri and or higher (meth)acryloyl esters include trimethylol propane tri(meth)acrylate, ethoxylated glycerin tri(meth)acrylate, ethoxylated trimethylolpropane tri(meth)acrylate, ditrimethylol propane tetra(meth)acrylate, pentaerythritol tri(meth)acrylate, pentaerythritol tetra(meth)acrylate, propoxylated pentaerythritol tetra(meth)acrylate, ethoxylated pentaerythritol tetra(meth)acrylate, dipentaerythritol hexa(meth)acrylate, and ethoxylated isocyanuric acid tri(meth)acrylates.

Urethane(meth)acrylates, useful in the present invention, have at least two or more acryloyl or methacryloyl groups and a urethane group. Examples include urethanes based on aliphatic, aromatic, polyester, and polyether polyols and aliphatic, aromatic, polyester, and polyether diisocyanates capped with (meth)acrylate endgroups. Isocyanate prepolymers can also be used in place of the polyol/ diisocyanate core. Epoxy (meth)acrylates and epoxy urethane (meth)acrylates, useful in the present invention, have at least two or more acryloyl or methacryloyl groups and, optionally, a urethane group. Examples include epoxy (meth)acrylates based on aliphatic or aromatic epoxy prepolymers capped with (meth)acrylate endgroups. A aliphatic or aromatic urethane spacer can be optionally inserted between the epoxy and the (meth)acrylate endgroup(s). Acrylated polyester oligomers, useful in the present invention, have at least two or more acryloyl or methacryloyl groups and a polyester core. Acrylated polyether oligomers, useful in the present invention, have at least two or more acryloyl or methacryloyl groups and a polyether core. Acrylated acrylate oligomers, useful in the present invention, have at least two or more acryloyl or methacryloyl groups and a polyacrylic core. These reactive urethanes, epoxies, polyesters, polyethers and acrylics are available from several suppliers including BASF Corporation, Bayer MaterialScience, Bomar Specialties Co, Cognis Corporation, Cytec Industries Inc, DSM NeoResins, Eternal Chemical Co, Ltd, IGM Resins, Rahn AG, Sartomer USA, LLC, and SI Group, Inc.

In addition to the above-described (meth)acrylate-based polymerizable monomers, other polymerizable monomers, oligomers or polymers of monomers which contain at least one free radical polymerizable group in the molecule may be used without any limitations in the curable gel. These monomers may contain an acidic group to improve adhesion.

A compound having at least one free radical polymerizable group includes not only a single component but also a mixture of polymerizable monomers. Thus combinations of two or more materials containing free radical polymerizable groups may be used in combination.

The gels also contain a photoinitiator. Examples of these include include benzyl ketones, monomeric hydroxyl ketones, polymeric hydroxyl ketones, .alpha.-amino ketones, acyl phosphine oxides, metallocenes, benzophenone, benzophenone derivatives, and the like. Specific examples include 1-hydroxy-cyclohexylphenylketone, benzophenone, 2-benzyl-2 (dimethylamino)-1-(4-(4-morphorlinyl)phenyl)-1-butanone, 2-methyl-1-(4-methylthio)phenyl-2-(4-morphorlinyl)-1-propanone, diphenyl-(2,4,6-trimethylbenzoyl) phosphine oxide, phenyl bis(2,4,6-trimethylbenzoyl) phosphine oxide, benzyl-dimethylketal, isopropylthioxanthone, and mixtures thereof.

Photo accelerators such as aliphatic or aromatic amines may also be included in the gel as well as fillers, inhibitors, plasticizers, non-reactive polymers, and adhesion promoters.

By the term "gel," we mean a radiation-curable composition comprising photoinitiator, ethylenically unsaturated monomers and/or oligomers, having a viscosity suitable for coating natural or artificial nails, or forming artificial nails and extensions, as well as adorning such nails.

A different viscosity range is preferred for each of these applications. Typical viscosities can range widely, from 2 to 500 poise, depending on the application. For building artificial nails, viscosities between 20 and 500 poise are commercially available. For coatings, less than 25 poise is typical.

There are many possible embodiments of the gel. In some embodiments the gel is comprised of 70-80% by weight an aliphatic polyester based urethane diacrylate oligomer, 20-30% by weight glycol HEMA-methacrylate (ethylene glycol dimethacrylate). 3-5% by weight hydroxycyclohexyl phenyl ketone, and 3-5% by weight benzophenone. In certain other embodiments the gel is comprised of 60-70% by weight an aliphatic polyester based urethane diacrylate oligomer, 5-10% by weight 2-hydroxyethyl methacrylate (HEMA), 5-10% by weight isobornyl methacrylate, and up to 1% by weight hydroxycyclohexyl phenyl ketone. Another embodiment of the gel is comprised of 50-60% by weight an aliphatic polyester based urethane diacrylate oligomer, 15-20% by weight HEMA, 15-20% by weight hydroxypropyl methacrylate, and up to 1% by weight hydroxycyclohexyl phenyl ketone.

The pigment concentrates which are used in the invention generally contain 10-50% pigment which may be dispersed in an organic liquid comprised of one or more chemicals selected from solvents, ethylenically unsaturated monomers, and ethylenically unsaturated oligomers. The organic liquid may also comprise non-reactive polymer, filler, and dispersant. For example, the organic liquid may comprise nitrocellulose. The organic liquid has one continuous phase whereas the pigment is a discontinuous phase of the pigment concentrate. Examples of suitable solvents are butyl acetate, ethyl acetate, isopropanol, xylene, toluene, acetone, and methyl ethyl ketone. Examples of ethylenically unsaturated monomers are (meth)acrylic esters, and examples of ethylenically unsaturated oligomers are urethane (meth)acrylates. The concentrates may be dispersed in the same UV-curable monomers and/or oligomers as used in the gel formulation by any means, for example by shearing of the pigment directly into the organic liquid. In one embodiment the organic liquid comprises ethyl acetate, butyl acetate, and nitrocellulose.

Suitable pigments which can be incorporated into the concentrates include barium, calcium and aluminum lakes, iron oxides, chromates, molybdates, cadmiums, metallic or mixed metallic oxides, talcs, carmine, titanium dioxide, chromium hydroxides, ferric ferrocyanide, ultramarines, titanium dioxide coated mica platelets, and/or bismuth oxychlorides, Preferred pigments include D&C Black No. 2, D&C Black No. 3., FD&C Blue No. 1, D&C Blue No. 4, D&C Brown No. 1, FD&C Green No. 3, D&C Green No. 5, D&C Green No. 6, D&C Green No. 8. D&C Orange No. 4, D&C Orange No. 5, D&C Orange No. 10, D&C Orange No. 11,
FD&C Red No. 4., D&C Red No. 6, D&C Red No. 7, D&C Red No. 17, D&C Red No. 21, D&C Red No. 22, D&C Red No. 27, D&C Red No. 28, D&C Red No. 30. D&C Red No. 31, D&C Red No. 33, D&C Red No. 34, D&C Red No. 36, FD&C Red No. 40, D&C Violet No. 2, Ext. D&C Violet No. 2, FD&C Yellow No. 5, FD&C Yellow No. 6, D&C Yellow No. 7, Ext. D&C Yellow No. 7, D&C Yellow No. 8, D&C Yellow No. 10, D&C Yellow No. 11, as well as others listed on the FDA color additives website, and Annex IV of the Cosmetic Directive 76/768/EEC, Coloring Agents Permitted in Cosmetics.

These pigments are homogenously dispersed into the concentrate and then the concentrate is incorporated into the final gel product by blending without the need for high shear processing. The ratio of pigment concentrate to gel composition is preferably equal to or less than 1:2.

The use of high color pigment content in these final gels (>0.4 pph) can reduce the ability to cure thick films and thus thinner coats of the resulting gel are preferred. To accomplish this, gels with lower viscosity than those typically used as builder gels are preferred, however high viscosity gels can also be used. Lower viscosity gels are preferred since their application properties are similar to standard nail polishes. Gel viscosities as measured at 25 °C, 1/2 sec shear, on a TA Instruments AR500 Rheometer of around 3000 poise are considered high viscosities whereas gel viscosities of <25 poise are preferred.

In some embodiments the pigment concentrate can be sold separately from the gel so that the consumer or nail technician can mix them together before application.

### EXAMPLES Example 1

To 49.6 grams of UV-curable gel comprised of 58% by weight an aliphatic polyester based urethane diacrylate oligomer, 20% by weight hydroxyethyl methacrylate, 20% by weight hydroxypropyl methacrylate, and 2% by weight hydroxycyclohexyl phenyl ketone was added sequentially, with hand stirring, three pigment concentrate pastes. Each pigment concentrate paste was a dispersion of pigment in an organic liquid composed of butyl acetate solvent (30.0% - 40.0%), ethyl acetate solvent (20.0% - 30.0%), nitrocellulose (10.0% - 20.0%), and isopropyl alcohol solvent 1.0% - 5.0%. The pigments were Ti02, D&C Red #6, and D&C Red #7 Light, and the amounts of dispersion added were 0.1, 5.9, and 2.8 grams, respectively.

### Example 2 (Comparative)

To 49.6 grams of the same UV-curable gel described in Example 1 were added 6 grams of OPI Big Apple Red Nail Polish with hand stirring.

In order to test whether the method of the invention was successful in matching the color and color density of the nail polish itself, the mixtures from Example 1, Comparative Example 1 and OPI Big Apple Red nail polish were each coated on 25 mm X 75 mm slides to give a 1 inch X 25 mm square. Different numbers of coats and coating weights were used. Coatings made with Example 1 and Comparative Example 1 were cured under UV lights for three minutes prior to applying a subsequent coat and again after the final coat. The nail polish example was dried for 30 min in between coats. A group of experts were then asked to rate the samples according to color density. Table 1 gives the results.

**Table 1**

| | Comparative Example 1 | | | | | OPI Big Apple Red Nail Polish | | | | | Example 1 | | | | |
|---|---|---|---|---|---|---|---|---|---|---|---|---|---|---|---|
| | Number Of Coats/Coating Weight (mg) | | | | | Number Of Coats/Coating Weight (mg) | | | | | Number Of Coats/Coating Weight (mg) | | | | |
| | 1/69 | 1/107 | 2/172 | 3/255 | 4/320 | | | 1/71 | 2/141 | | | 1/68 | 1/82 | 2/147 | |
| | Rating* | | | | | | | | | | | | | | |
| Rater 1 | 1 | 2 | 3 | 5 | 8 | | | 4 | 9 | | | 7 | 6 | 10 | |
| Rater 2 | 1 | 2 | 3 | 5 | 8 | | | 4 | 9 | | | 6 | 7 | 10 | |
| Rater 3 | 1 | 2 | 3 | 6 | 8 | | | 4 | 9 | | | 5 | 7 | 10 | |
| Rater 4 | 1 | 2 | 3 | 5 | 7 | | | 4 | 10 | | | 6 | 8 | 9 | |
| Rater 5 | 1 | 2 | 3 | 7 | 8 | | | 4 | 9 | | | 5 | 6 | 10 | |
| Average | 1 | 2 | 3 | 5.6 | 7.8 | | | 4 | 9.2 | | | 5.8 | 6.8 | 9.8 | |

| | | | | | | | | | | | | | | | |
|---|---|---|---|---|---|---|---|---|---|---|---|---|---|---|---|
| *Rating Scale - 1 = lowest color density, 10=highest color density | | | | | | | | | | | | | | | |

The ratings demonstrate that color density is significantly lower at any given number of coats or coating weight using the method of Comparative Example 1 as compared to Example 1. Significantly thicker coats of the comparative example were required in order to match either the initial nail polish color or the mixture from Example 1. Thus, an improved artificial nail gel material can be made from the method of the invention compared to that made via the comparative method.

## Claims

1. A method of preparing colored UV-curable compositions useful for adornment of natural and artificial nails and artificial nail extensions comprising dispersing a pigment in an organic liquid to form a pigment concentrate, the organic liquid comprising one or more organic chemicals selected from solvents, ethylenically unsaturated monomers, and ethylenically unsaturated oligomers,; and mixing the dispersed pigment concentrate with a radiation-curable nail gel composition comprising one or more ethylenically unsaturated monomers, one or more ethylenically unsaturated oligomers, or mixtures thereof, and a photoinitiator, wherein
the concentration of pigment in the pigment concentrate is 10 to 50%.

2. The method of claim 1 wherein organic liquid is a non-reactive solvent.

3. The method of claim 1 wherein organic liquid comprises one or more non-reactive solvents selected from butyl acetate, ethyl acetate, isopropanol, xylene, toluene, acetone, and methyl ethyl ketone.

4. The method of claim 1 wherein organic liquid comprises one or more chemicals selected from mono-, di-, tri-, and tetra-functional ethylenically unsaturated monomers and oligomers.

5. The method of claim 1 wherein organic liquid comprises one or more chemicals selected from (meth)acrylic monomers and oligomers.

6. The method of claim 1 wherein the gel composition comprises a mono-, di-, tri-, or tetra-functional acrylic or methacrylic monomer.

7. The method of claim 1 wherein the gel composition comprises a polyfunctional polyurethane (meth)acrylate oligomer.

8. The method of claim 1 wherein the gel composition comprises monomer selected from hydroxyethyl (meth)acrylate, hydroxypropyl (meth)acrylate, trimethylolpropane tri(meth)acrylate and isobornyl (meth) acrylate.

9. The method of claim 1 wherein the gel composition comprises aliphatic polyester based urethane diacrylate oligomer.

10. The method of claim 1 wherein the artificial nail gel comprising the pigment concentrate has a viscosity of below 2.5 Pa.s (25 poise).

11. The method of claim 1 wherein the ratio by weight of pigment concentrate to radiation-curable nail gel composition is preferably equal to or less than 1:2.

12. The method of claim 1 wherein the organic liquid comprises nitrocellulose.

## Patentansprüche

1. Verfahren zur Herstellung gefärbter UV-härtender Zusammensetzungen, die zum Verzieren natürlicher und künstlicher Fingernägel sowie künstlicher Nagelverlängerungen nützlich sind, umfassend Dispergieren eines Pigments in einer organischen Flüssigkeit, um ein Pigmentkonzentrat zu bilden, wobei die organische Flüssigkeit umfasst: eine oder mehrere Chemikalien, die aus der nachfolgenden Gruppe gewählt sind: Lösemittel, ethylenisch ungesättigte Monomere und ethylenisch ungesättigte Oligomere; und Mischen des dispergierten Pigmentkonzentrats mit einer strahlungshärtbaren Nagelgelzusammensetzung, umfassend mindestens ein ethylenisch ungesättigtes Monomer, mindestens ein ethylenisch ungesättigtes Oligomer oder Mischungen davon und einen Photoinitiatior, wobei
die Konzentration des Pigments im Pigmentkonzentrat 10 - 50 % beträgt.

2. Verfahren nach Anspruch 1, wobei die organische Flüssigkeit ein nicht reaktives Lösemittel ist.

3. Verfahren nach Anspruch 1, wobei die organische Flüssigkeit ein oder mehrere nicht reaktive Lösemittel umfasst, die aus der nachfolgenden Gruppe gewählt sind: Butylacetat, Ethylacetat, Isopropanol, Xylol, Toluol, Aceton und Methylethylketon.

4. Verfahren nach Anspruch 1, wobei die organische Flüssigkeit eine oder mehrere Chemikalien umfasst, die aus den mono-, di-, tri- und tetrafunktionell ethylenisch ungesättigten Mono- und Oligomeren gewählt sind.

5. Verfahren nach Anspruch 1, wobei die organische Flüssigkeit eine oder mehrere Chemikalien umfasst, die aus den (Meth-)Acrylmonomeren und -oligomeren gewählt sind.

6. Verfahren nach Anspruch 1, wobei die Gelzusammensetzung ein mono-, di-, tri- oder tetrafunktionelles Acryl- oder Methacrylmonomer umfasst.

7. Verfahren nach Anspruch 1, wobei die Gelzusammensetzung ein polyfunktionelles Polyurethan(meth)acrylatoligomer umfasst.

8. Verfahren nach Anspruch 1, wobei die Gelzusammensetzung ein aus der nachfolgenden Gruppe gewähltes Monomer umfasst: Hydroxyethyl(meth)acrylat, Hydroxypropyl (meth)acrylat, Trimethylolpropantri(meth)acrylat und Isobornyl(meth)acrylat.

9. Verfahren nach Anspruch 1, wobei die Gelzusammensetzung ein Urethandiacrylatoligomer auf aliphatischer Polyesterbasis umfasst.

10. Verfahren nach Anspruch 1, wobei das das Pigmentkonzentrat umfassende künstliche Nagelgel eine Viskosität kleiner 2,5 Pa.s (25 Poise) aufweist.

11. Verfahren nach Anspruch 1, wobei das Gewichtsverhältnis von Pigmentkonzentrat zu strahlungshärtender Nagelgelzusammensetzung vorzugsweise kleiner oder gleich 1 : 2 ist.

12. Verfahren nach Anspruch 1, wobei die organische Flüssigkeit Nitrocellulose umfasst.

## Revendications

1. Procédé de préparation de compositions durcissables aux UV colorées utiles pour la décoration d'ongles naturels et artificiels et d'extensions artificielles d'ongles, comprenant la dispersion d'un pigment dans un liquide organique pour former un concentré de pigment, le liquide organique comprenant un ou plusieurs produits chimiques organiques choisis parmi les solvants, les monomères à insaturation éthylénique, et les oligomères à insaturation éthylénique ; et le mélange du concentré de pigment dispersé avec une composition de gel pour ongles durcissable par irradiation comprenant un ou plusieurs monomères à insaturation éthylénique, un ou plusieurs oligomères à insaturation éthylénique, ou leurs mélanges, et un photoamorceur, dans lequel
la concentration de pigment dans le concentré de pigment est de 10 à 50 %.

2. Procédé selon la revendication 1, dans lequel le liquide organique est un solvant non réactif.

3. Procédé selon la revendication 1, dans lequel le liquide organique comprend un ou plusieurs solvants non réactifs choisis parmi l'acétate de butyle, l'acétate d'éthyle, l'isopropanol, le xylène, le toluène, l'acétone, et la méthyléthylcétone.

4. Procédé selon la revendication 1, dans lequel le liquide organique comprend un ou plusieurs produits chimiques choisis parmi les monomères et oligomères à insaturation éthylénique mono-, di-, tri- et tétra-fonctionnels.

5. Procédé selon la revendication 1, dans lequel le liquide organique comprend un ou plusieurs produits chimiques choisis parmi les monomères et oligomères (méth)acryliques.

6. Procédé selon la revendication 1, dans lequel la composition de gel comprend un monomère acrylique ou méthacrylique mono-, di-, tri- ou tétra-fonctionnel.

7. Procédé selon la revendication 1, dans lequel la composition de gel comprend un oligomère de (méth)acrylate de polyuréthane polyfonctionnel.

8. Procédé selon la revendication 1, dans lequel la composition de gel comprend un monomère choisi parmi le (méth)acrylate d'hydroxyéthyle, le (méth)acrylate d'hydroxypropyle, le tri(méth)acrylate de triméthylolpropane et le (méth)acrylate d'isobornyle.

9. Procédé selon la revendication 1, dans lequel la composition de gel comprend un oligomère de diacrylate d'uréthane à base de polyester aliphatique.

10. Procédé selon la revendication 1, dans lequel le gel pour ongles artificiels comprenant le concentré de pigment a une viscosité inférieure à 2,5 Pa.s (25 poises).

11. Procédé selon la revendication 1, dans lequel le rapport en poids du concentré de pigment à la composition de gel pour ongles durcissable par irradiation est de préférence égal ou inférieur à 1/2.

12. Procédé selon la revendication 1, dans lequel le liquide organique comprend de la nitrocellulose.
